# EUROPEAN PATENT SPECIFICATION

(11) **EP 1 213 014 B1**
(45) Date of publication and mention of the grant of the patent: **20.04.2005**
(21) Application number: 01128075.7
(22) Date of filing: 27.11.2001
(51) Int. Cl.: A61K 9/22

(54) **Process and system for uniform release drug delivery**
Verfahren und System zur gleichmä igen Arzneistoffabgabe
Procédé et système pour la délivrance uniforme d' un principe actif

(30) Priority: 07.12.2000 US 251996 P
(43) Date of publication of application: 12.06.2002
(73) Proprietor: Warner-Lambert Company LLC, Morris Plains, New Jersey 07950 (US)
(72) Inventor: Fessehaie, Mebrahtu, Mineola, New York 11501 (US); Ghebre-Sellassie, Isaac, Morris Plains, New Jersey 07950 (US); Mollan, Matthew Joseph Jr., Succasunna, New Jersey 07876 (US); Mayassi, Monzer Michael, Somerset, New Jersey 08873 (US); Woldegaber, Haimanot, Dover, New Jersey 07801 (US); Dyar, Stephen Craig, Brighton, Michigan 48116 (US)
(74) Representative: Tesch, Rudolf, Dr.

(56) References cited:
- EP-A- 0 891 769
- WO-A-92/05776
- WO-A-95/22962
- WO-A-99/08660

## Description

### FIELD OF THE INVENTION

The present invention relates to a co-extruded, controlled-release pharmaceutical dosage form and a method for manufacturing such a dosage form.

### BACKGROUND OF THE INVENTION

A common objective of convenience in medical treatment is to provide a pharmaceutically-active agent in a matrix and/or in a fully or partially surrounding coating which controls the release of the diffusion of the pharmaceutically-active agent into surrounding bodily fluid by chemical and/or mechanical means, thereby providing uniform dosing over a prolonged period following a single administration.

Among such prior proposals are those disclosed in the following patent publications:

### United States Patent Number 3,113,076 (Jacobs)

Jacobs describes a controlled-release medicament, in the form of a tablet, comprising "a substantially uniform mixture" of an active ingredient and a pharmaceutical vehicle. The tablet has at least one aperture to control the inner and outer surface areas of the medicament for controlling release characteristics. The tablet may be coated to further slow release of the active ingredient. The tablet is formed by pressing, molding, and/or extruding.

### United States Patent Number 4,755,180 (Ayer, et al)

Ayer, et al, describes a polymeric-coated osmotic dosage form with an exit hole (or other passageway) for direct release of an active ingredient from the interior of the polymer coating. The dosage form further comprises a semipermeable wall (polymeric coat impermeable to the active ingredient) surrounding a core of active ingredient combined with an "osmotically effective solute" which is said to control osmotic migration of fluid and active ingredient into and out of the semipermeable membrane enclosure.

### United States Patent Number 4,816,262 (McMullen)

McMullen describes a controlled-release tablet having a core of active drug substance coated by a hydrophobic material. A hole of varying dimension through the center of the tablet is said to provide a controllable mechanism for release of the active ingredient. The outer coating is applied to the tablet by dip coating.

### United States Patent Number 4,839,177 (Columbo et al)

Columbo et al describes a system for controlled-rate release comprising a "deposit-core" of a defined geometric shape containing an active ingredient adjacent to a water impermeable support platform partially coating the "deposit core." A swellable polymeric material, combined in the "deposit core" with the active ingredient, controls the intake of water by swelling. The coating does not allow release of the active ingredient. All of the active ingredient is released from the exposed portion of the "deposit core" upon swelling of the swellable polymer with which it is combined. The support platform is formed by compression of polymeric material, immersion in a solution of polymeric material, or by spraying the solution of polymeric material onto the core.

### United States Patent Number 5,681,568 (Goldin et al)

Goldin et al describes a microporous membrane for delivery of macromolecules to a therapeutic target. The membrane contains a microporous underlayment, a microskin, and a macromolecule(s) in the form of a hollow fiber. Co-extrusion is used to form the hollow fiber. The fibrous material is extruded as the outer layer, and a polymer solution is extruded inside of the fibrous material such that a hollow, annular form is obtained.

### European Patent Application Number 0 891 769 A1

This European patent application discloses a method in which a co-extrusion device is used to produce a controlled-release pharmaceutical dosage form. The outer extruded coating apparently completely surrounds a pharmaceutical agent-containing core.

Co-extrusion itself is also used in other applications, as shown, for example, in United States Patent Number 5,417,992, which discloses the making of food products wherein a cheese filling is surrounded by a dough or puffed outer composition.

WO 92/05 776 and WO 95/22 962 describe a controlled release composition wherein the surface areas of the core remain constant upon erosion. However they fail to say that the core is a glassy extrudate matrix.

Notwithstanding the above-referenced prior information, the present inventors have developed what they believe to be a simpler and possibly more consistent controlled-release dosage form, as well as an improved method of making such a dosage form.

### SUMMARY OF THE INVENTION

In accordance with the present invention, a controlled-release drug dosage form comprises a central core, which includes a pharmaceutical agent, in a carrier matrix, typically a controlled-release composition. The core has two exposed opposite end surfaces and a peripheral surface at an outer edge of the core extending between the end surfaces. That peripheral surface is surrounded by a diffusion-limiting sleeve or coating. The diffusion-limiting sleeve is substantially impervious to water or bodily fluids, so that the release rate of the pharmaceutical agent in the central core is governed entirely by erosion or dissolution at the exposed surfaces. The surface area of each of the exposed faces remains substantially constant through the drug delivery period. Generally, the core and sleeve are cylindrical.

The pharmaceutical dosage form of present invention is formed by co-extrusion of said central core and said sleeve resulting in said central core composed of a glassy matrix.

The present invention also comprises a method of making a controlled-release dosage form as a single or multiple unit dose by coextruding composite products comprising a central core of pharmaceutical agent of low or high water solubility, preferably in a matrix of a controlled-release composition, and a surrounding sleeve of a diffusion-limiting composition. The co-extrudate is sliced axially to produce a plurality of dosage forms each comprising a central core in which the pharmaceutical agent core has two oppositely faced exposed surfaces and is surrounded by the diffusion-limiting sleeve of outer co-extrudate. Preferably, the oppositely faced exposed surfaces are parallel to each other.

As used herein, controlled-release implies the controlled delivery of a drug substance at specific sites within the gastrointestinal tract of a mammal, and includes immediate, extended, pulsatile, enteric, and colonic release.

Controlled-release of other active ingredients and veterinary products may also be effected by incorporation of such active ingredients in a core and surrounding sleeve product analogous to that otherwise disclosed herein.

For a better understanding of the present invention, reference may be made to the following detailed description and the appended claims, taken together with the accompanying drawings.

### BRIEF DESCRIPTION OF THE DRAWINGS

Figure 1 is a schematic view of a co-extrusion apparatus that can be used in the method of the present invention.
Figure 2 is a cross-sectional view of the common die 17 of the co-extrusion apparatus shown in Figure 1.
Figure 3 is a perspective view of a pharmaceutical dosage form of the present invention.
Figures 4 and 5 are schematic views of barrel and screw designs for use in extruders useful in the method of the present invention.
Figure 6 is a graphical representation of the linear release rate of a drug substance from an exemplary formulation made according to the present invention.

### DETAILED DESCRIPTION OF THE INVENTION

Soluble controlled-release dosage forms of pellets, disks, etc., by which pharmaceutical agents (or active ingredients such as pesticides or herbicides) are controllably released for dissolution to surrounding fluids are well-known. Typically, the pharmaceutically-active agent is disposed in a matrix material and/or coating material which controls the delivery of the pharmaceutically-active agent by controlling either the access of the surrounding bodily fluids to the pharmaceutically-active agent, or controls the release outwardly from the matrix material or coating of the pharmaceutically-active agent.

The present co-extruded controlled-release dosage form may utilize any pharmaceutically-active agent, preferably those active agents which are suitable for human consumption, and controlled-release matrix material compositions in which such agents (or other active ingredients) may be included. In addition, the agent or ingredient is embedded in an erodible matrix. Further, the present invention encompasses essentially any suitably active ingredient, which may or may not be water soluble, that can be formulated into a solid dosage form for desired release kinetics, such as zero-order controlled-release delivery. Examples of specific active ingredients which may be useful and, preferably, are suitable for human consumption, are listed below:
1. Antipyretic, analgesic and anti-inflammatory agents, such as indomethacin, aspirin, diclofenac sodium, ketoprofen, ibuprofen, mefenamic acid, dexamethasone, hydrocortisone, prednisolone, acetaminophen, phenylbutazone, flufenamic acid, sodium salicylate, tramadol hydochloride, oxaprozin, and etodolac.
2. Antiulcer agents, such as omeprazole, cimetidine, lansoprazole, ranitidine hydrochloride, famotidine, and nazatidine.
3. Coronary vasodilators, such as nifedipine, isosorbide dinitrate, diltizem hydrochloride, dipyridamole, isosorbide mononitrate, verapamil, nicardipine, nifedipine, and nitroglycerin.
4. Peripheral vasodilators, such as sildenafil citrate, cinepazide maleate, cyclandelate, and pentoxiphylline.
5. Antibiotics, such as ampicillin, amoxicillin, cefalexin, clarithromycin, cefuroxime axetil, cefropzil, erythromycin ethyl succinate, bacampicillin hydrochloride, minocycline hydrochloride, chloramphenicol, tetracycline, and erythromycin.
6. Synthetic antimicrobial agents, such as nalidixic acid, enoxacin, cinoxacin, levofloxacin, ofloxacin, norfloxacin, ciprofloxacin hydrochloride, and sulfamethoxazole-trimoethoprim.
7. Antispasmotic agents, such as propantheline bromide, atropine sulfate, and scopolamine.
8. Antitussive and antiasthmatic agents, such as theophylline, aminophylline, codeine phosphate, dextromethorphan, ephedrine hydrochloride, and noscapine.
9. Bronchodilators, such as salbutamol sulfate, pirbuterol hydrochloride, bitolterol mesilate, clenbuterol hydrochloride, terbutaline sulfate, mabuterol hydrochloride, fenoterol hydrobromide, and methoxyphenamine hydrochloride.
10. Diuretics, such as furosemide, acetazolamide, trichlormethiazide, cyclothiazide, hydrochlorothiazide, hydroflumethiazide, spironolactone, and triamterene.
11. Muscle relaxants, such as tolperisone hydrochloride, eperisone hydrochloride, tizanidine hydrochloride, mephenesin, chlorzoxazone, phenprobamate, methocarbamol, baclofen, and dantrolene sodium.
12. Cerebral metabolism improving agents, such as meclofenate hydrochloride.
13. Tranquilizers, such as oxazolam, diazepam, temazepam, meprobamate, nitrazepam, chlordiazepoxide, sulpiride, clocapramine hydrochloride, zotepine, chlorpromazine, and haloperidol.
14. Beta-Blockers, such as pindolol, propranolol hydrochloride, metoprolol tartrate, labetalol hydrochloride, oxprenolol hydrochloride, acebutolol hydrochloride, metoprolol succinate, bufetolol hydrochloride, alprenolol hydrochloride, and nadolol.
15. Antiarrhythmic agents, such as procainamide hydrochloride, disopyramide, quinidine sulfate, propafenone hydrochloride, and mexiletine hydrochloride. Antigout agents, such as allopurinol, probenecide, colchicine, warfarin sodium, and sulfinpyrazone.
16. Antigout agents, such as allopurinol, probenecide, colchicine, warfarin sodium, and sulfinpyrazone.
17. Anticoagulants, such as ticlopidine hydrochloride, dicoumarol, and warfarin potassium.
18. Antiepileptics, such as gabapentin, pregabalin, phenytoin, divalproex sodium, sodium valproate, and metharbital.
19. Antihistaminics, such as loratadine, cetirizine hydrochloride, chlorpheniramine maleate, fexofenade hydrochloride, celmastine fumarate, and cyproheptadine hydrochloride.
20. Antiemetics, such as difenidol hydrochloride, methoclopramide, and trimebutine maleate.
21. Antihypertensive agents, such as methyldopa, prazosin hydrochloride, bunazosin hydrochloride, clonidine hydrochloride, budralasine, bisporolol fumarate, hydrochlorothiazide, terazosin hydrochloride, and urapidil.
22. Sympathomimetric agents, such as dihydroergotamine mesilate, isoproterenol hydrochloride, and etilefrine hydrochloride.
23. Expectorants, such as bromhexine hydrochloride, carbocysteine and cysteine methyl ester hydrochloride
24. Oral antidiabetic agents, such as glubenclamide, glimepiride, gliprizide, metformin hydrochloride, troglitazone, rosiglitazone, pioglitazone, tolbutamide, and glymidine sodium.
25. Iron preparations, such as ferrous sulfate and dried iron sulfate.
26. Vitamins, such as vitamin B₁₂, vitamin B₆, vitamin C, and folic acid.
27. Therapeutic agents for pollakiuria, such as flavoxate hydrochloride, oxybutynin hydrochloride, and terodiline hydrochloride.
28. Angiotensin converting enzyme inhibitors, such as enalapril maleate, enalaprilat, fosinopril sodium, alacepril, lisinopril, quinapril hydrochloride, ramipril, and delapril hydrochloride.
29. Other types of active ingredients, that can be formulated according to this invention include acetohexamide, ajamaline, alendronate sodium, amlodipine besylate, amylobarbitone, atorvastatin calcium, simvastatin, pravastatin, fluvastatin, rosuvastatin, bendrofluozide, benzbromarone, benzoate, benzylbenzoate, betametharzone, paroxetine hydrochloride, buproprion hydrochloride, buspirone hydrochloride, chloramphenicol, chloropropamide, chlorthalidone, clofibrate, conjugated estrogens, corticosteroids, diazepam, dicumerol, digitoxin, digoxin, dihydroxypropyltheophylline, diltiazem hydrochloride, doxazosin mesylate, ergot alkaloids, ethotion, felodipine, fluoxetine hydrochloride, fluconazole, fluvastatin sodium, frusemide, glutethimide, griseofulvin, hydrochlorothiazide, hydrocortisone, hydroflumethiazide, hydroquinone, hydroyalkylxanthines, indomethacin, isoxsuprine hydrochloride, ketoprofen, khellin, levothyroxine sodium, losartan potassium, lovastatin, meprobamate, nabilone, nefazodone hydrochloride, nicotinamide, nifedipine, nitrofurantoin, novalgin, nystatin, papaverine, paracetamol, phenylbutazone, phenobarbitone, pravastin sodium, prednisolone, prednisone, primadonel, reserpine, risperidone, romglizone, salicylic acid, salmeterol xinafoatte, sertraline hydrochloride, simvastatin, spironolactone, sulphabenzamide, sulphadiamadine, sulphamethoxydiazine, sulphamerazine, succinylsulphathiazole, sulphamethizole, sulphamethoxazole, sulphathiazole, sulphisoxazole, sumatriptain succinate, testosterone, tolazoline, tolbutamide, trifluoperazine, trimethoprim, valsartan, zolpidem tartrate and other water insoluble or water soluble active ingredients.

Typical matrix materials with which the pharmaceutically-active agents are combined may be selected from polyethylene glycol 400, polyvinylalcohol, polymethacrylates, cellulose acetate phthalate, polyvinylpyrrolidone, hydroxypropylcellulose phthalate, hydroxypropylmethylcellulose phthalate, hydroxyproplymethylcellulose acetate succinate, and polysorbate 80. Hydroxypropylcellulose and hydroxypropylmethylcellulose may also be incorporated in such matrix materials as well as polyethylene glycol of various molecular weights, ranging from 400 or below to 8000 and above. The matrix material may also include other common excipients, additives and solubility-controlling compounds, etc.

In accordance with the present invention, a core, including a pharmaceutically-active agent in a controlled-release matrix material, typically includes two exposed parallel planar end surfaces, with the edges of the core extending between those surfaces surrounded by a coating which is essentially water impervious. Controlled-release of the pharmaceutically-active agent from a dosage form of this configuration occurs only at the substantially constant cross-sectional area of the exposed surfaces. Alternatively, the exposed surfaces may be slanted, curved or angled, or a combination thereof.

Suitable materials for the essentially water-impervious outer coating of the dosage form of this invention include polymethacrylate or ethylcellulose. Plasticizers, such as dibutylsebacate, glycerine, and tributylcitrate, may also be incorporated in the coating formulation.

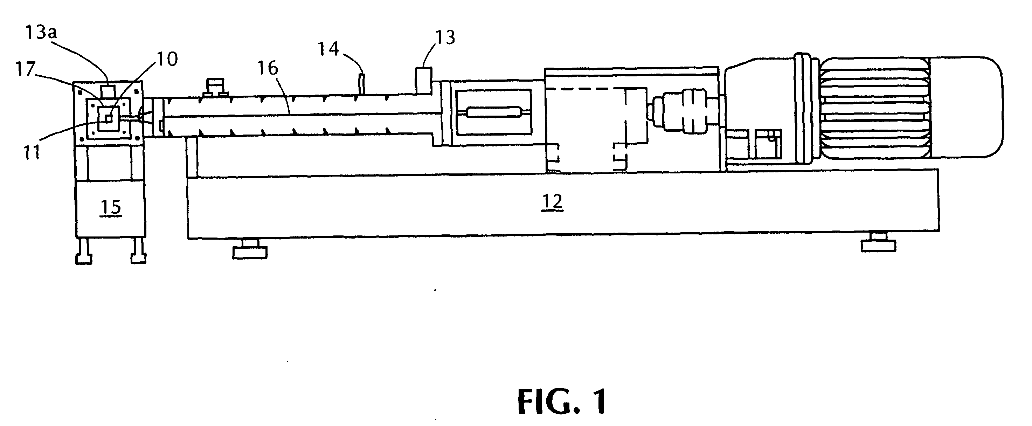

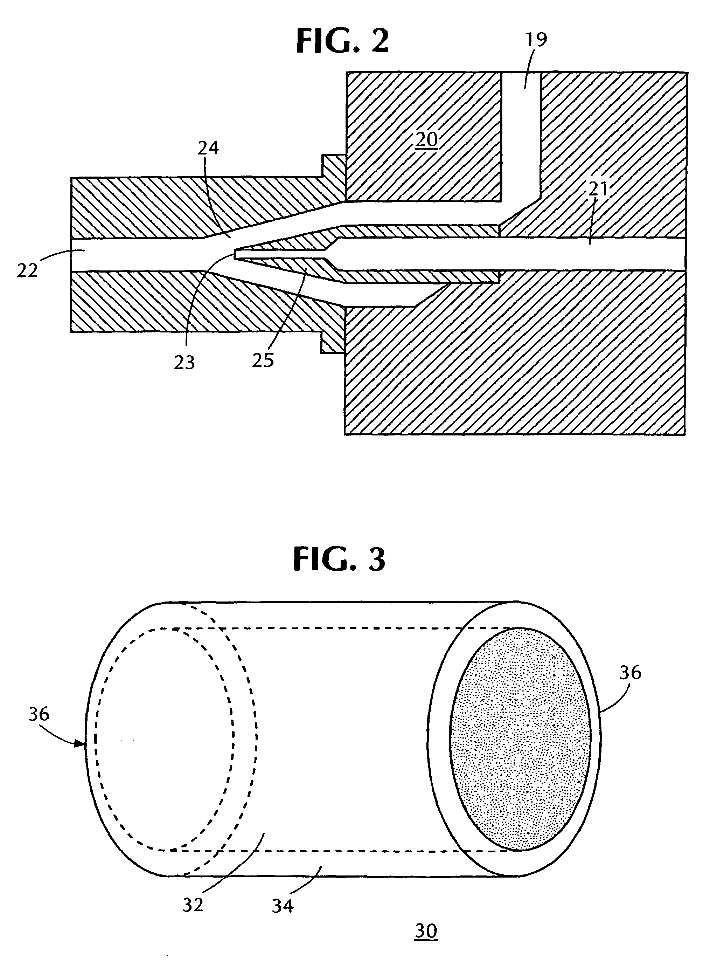

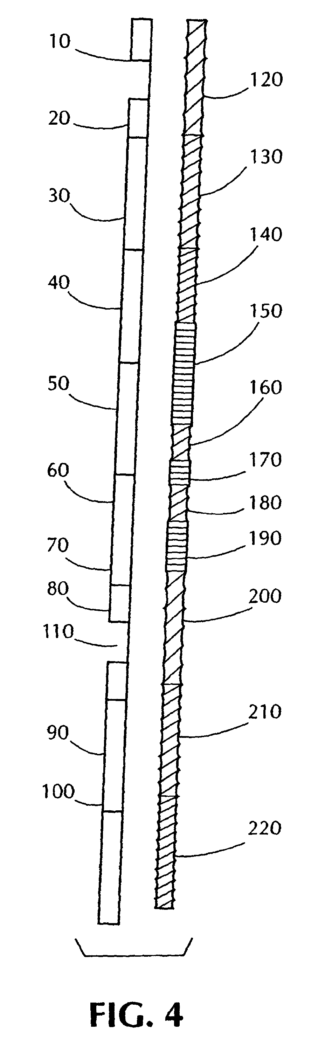

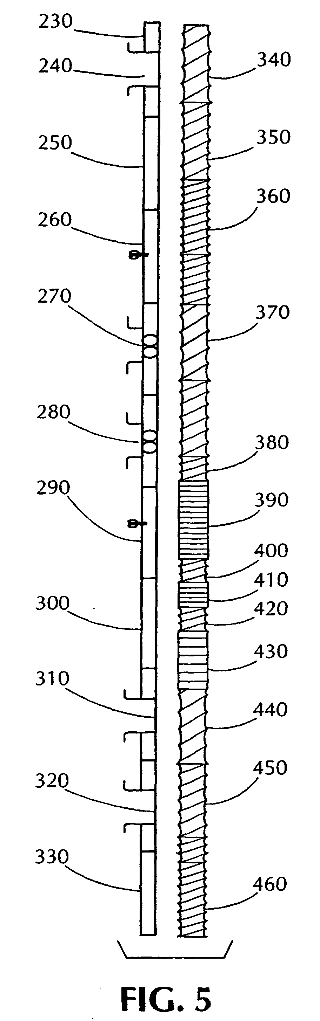

For the method of the present invention and for making the product of the present invention, an apparatus such as that shown schematically in Figures 1 and 2 may be used. This apparatus comprises a first extruder **12** which produces outer layer **11**, and a second extruder **15** for producing inner core **10** of a co-extrudate product. The components for the outer layer, including the pharmaceutically-active agent, are fed, for example, into first extruder **12** using appropriate powder and/or liquid feed ports **(13** and **14,** respectively), while the components for the inner core are fed into second extruder **15** through appropriate powder-feed ports **13a** and/or liquid-feed ports (not shown). Each extruder may be a twin-screw extruder having screws **16** (shown schematically) rotating in the same or opposite direction. Examples of such twin-screw extruder screws are described below with reference to Figure 4 and 5. To melt or soften the materials being mixed in and extruded from extruders **12** and **15**, heating elements (not shown) may be provided with sufficient capacity to melt or soften materials in the extruders, for example, to a temperature in the range of 30°C to 250°C, preferably 40°C to 200°C.

As shown in Figure 2, which is a bottom cross-sectional view of the mixing dies at the outlets of extruders **12** and **15,** the outlet passage **19** of first extruder **12**, and outlet passage **21** of second extruder **15**, intersect at a 90° angle in co-extrusion die **20**, which in turn leads downstream to central core outlet **23**, and a surrounding sleeve outlet **24**, and from there to die outlet **22**, from which is extruded the co-extrudate controlled-release drug delivery product. Surrounding sleeve outlet **24** extends entirely around the periphery of internal guide **25**.

The co-extrudate dosage form of the present invention may be sliced along diametrical planes to form a controlled-release tablet or pellet, such as the cylindrical pellet **30** shown in Figure 3. This slicing can occur either before or after hardening of the co-extrudate produced in the apparatus as shown in Figures 1 and 2. Typically, the slicing is accomplished by mechanical slicing or by laser slicing. This dosage form includes central core **32** containing the pharmaceutically-active agent, surrounded by an essentially water or bodily fluid impermeable outer coating sleeve **34**. Core **32**, at the ends of cylindrical pellet **30**, includes two parallel oppositely planar exposed end surfaces **36**, from which the pharmaceutically-active agent may be dissolved, eroded, diffused, or otherwise delivered substantially uniformly to the mammal to which it is administered. When the outer sleeve **34** is impermeable and nonerodible, at least within the time frame of drug release, exposed surfaces **36** provide a constant surface for drug release (i.e., the surface area from which the drug is released remains constant throughout the drug release period).

Controlled release cylindrical pellets can be introduced into a capsule, such as a gelatin capsule, soft gel capsule, hard gel capsule, and the like.

A pelleted dosage form, preferably having a total length less than the sum of the two diameters, more preferably one diameter of the pelleted dosage form, as shown in Figure 3, may be produced from the following exemplary materials, as shown in Table 2 and Table 2.

**Table 1.**

| Composition of Exemplary Coating Material | | | | |
|---|---|---|---|---|
| Function | Polymer | | Plasticizer | |
| | Name | Amount (%) | Name | Amount (%) |
| Coat | Eudragit RS | 95.0 | Triethylcitrate | 5.0 |
| Coat | Cellulose Acetate | 95.0 | Triethylcitrate | 5.0 |
| Coat | Ethylcellulose | 95.0 | Triethylcitrate | 5.0 |
| Coat | Cellulose Acetate | 80.0 | Triethylcitrate | 20.0 |

**Table 2.**

| Composition of Exemplary Core Material | | | | | | |
|---|---|---|---|---|---|---|
| Function | Polymer | | Plasticizer | | API | |
| | Name | Amount (%) | Name | Amount (%) | Name | Amount (%) |
| Core | PVP | 60.0 | PEG 400 | 10.0 | Sodium Phenytoin | 30 |
| Core | PVP | 43.5 | PEG 400 | 10.0 | Troglitazone | 43.5^{a} |
| API = Active pharmaceutical ingredient. | | | | | | |

| | | | | | | |
|---|---|---|---|---|---|---|
| ^{a} The last sample also includes Tween 80 at 3.0%. | | | | | | |

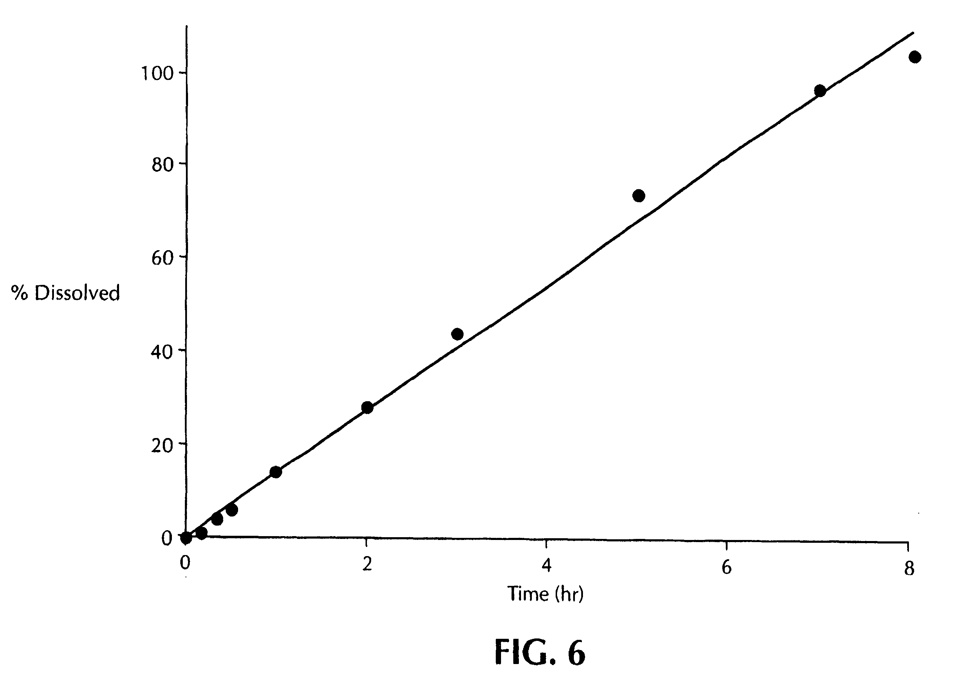

Pellets made in accordance with this invention comprise a core composed of a glassy matrix in which the pharmaceutically-active ingredient (API) (such as those set forth in Table 2 is dispersed molecularly or as particulate within a coating material, such as those set forth in Table 1. Because the core is typically composed of a glassy matrix, water and other fluids do not penetrate deeply into the exposed surfaces of the core. Therefore, the shape of the exposed core surfaces are substantially maintained during the entire course of drug release (as is the surface area, as described above), thereby providing an approximately linear release profile, as shown in Figure 6. Preferably, the release profile remains approximately linear until about 80% of total amount of API in the core has been released.

It will be recognized by those skilled in the art that a wide range of other materials may also be used as one or both of the core and outer sleeve materials in the co-extrudate product, and in the sliced co-extruded controlled-release dosage form produced therefrom. It will also be appreciated that the method of this invention may be implemented by those skilled in the art with a wide range of equipment and process parameter variables, all tailored to the production of the specific co-extrudate product. The preparation of co-extrudate controlled-release dosage forms is further illustrated by the following working examples. The examples are illustrative only, and are not to be construed as limiting the invention in any respect.

### EXAMPLE 1

Using the materials listed in Table 1, a formulation of troglitazone in PVP was prepared using a Leistritz Micro 18/GL 40D twin-screw extruder (Leistriz Inc, Somerville, NJ) with the screw design shown in Figure 4 (showing one barrel and one screw from a set composed of two barrels and two screws) to extrude the outer layer. The coating polymer material was introduced into the extruder via a K-Tron gravimetric feeder, and the coating plasticizer was introduced via a Masterflex peristaltic pump. The fed materials were mixed and heated as they were processed progressively through barrel segments **20, 30, 40, 50, 60, 70, 80, 90,** and **100** by screw segments **120, 130, 140, 150, 160, 170, 180, 190, 200, 210,** and **220** located within the barrel. The length of these screw segments was 90, 90, 60, 30, 30, 20, 30, 20, 90, 90, and 90 mm, respectively, and the internal diameters of the two barrels were 18 millimeters. The heater set point and processing temperature for zones 10-50, as shown in the left part of Figure 4, was 150°C, and was 145°C for zones 60-100. An injection port (not shown) is located in barrel segment **40** over screw segment **140**. A vent port **110** is located in barrel segment **80**. The Eudragit RS (polyethyl acrylate, methyl methacrylate, trimethylammonioethyl methacrylate chloride, in a 1:2:0.1 ratio) powder feed rate was 2.72 kg/h, while the liquid triethylcitrate (TEC) feed rate was 2.72 mL/min.

A Leistritz Micro 27/GL 40D twin-screw extruder with the screw design as shown in Figure 5 was used to extrude the core (comprised of materials such as those shown in Table 2). The materials (troglitazone/PVP) were introduced into the extruder via a K-Tron twin-screw feeder at port **240**, and the PEG 400/Tween 80 mixture was introduced via a Masterflex peristaltic pump via injection port located in barrel segment **260**. As the feed materials were mixed, they traversed the screws of Figure 5, after being fed through main feed throat **240** located in barrel segment **230**, through barrel segments **230, 250, 260, 270, 280, 290, 300, 310, 320,** and **330,** with inside barrel diameters of 27 mm, and screw segments **340, 350, 360, 370, 380, 390, 400, 410, 420, 430, 440, 450** and **460**. The length of the screw segments was 90, 90, 150, 180, 30, 30, 30, 30, 30, 10, 90, 90 and 120 mm, respectively. Injection ports were located in barrel segments **260** and **290** over screw segments **360** and **390**, respectively. In this example, nothing was added via the injection port located in barrel segment 290. Vent ports were located in barrel segments **310** and **320**. In this apparatus, additional injection feed ports may be located in barrel segments **270 and 280**. The temperature control set point and processing temperature for zones 230 and 250-280 was 150°C, and for zones 290-330 it was 145°C. Troglitazone was mixed uniformly with PVP polymer, and the troglitazone/PVP powder feed rate was 9.53 kg/h, while the liquid PEG 400/Tween 80 mixture feed rate was 24 mL/minute.

The extrudates of these respective extruders (the core and the coating) were combined in a common die with a 9:1 ratio (core:coat) which connected the two extruders at a 90° angle as shown in Figure 2. Other angular alignments, such as 180°, are also possible. A Dornier fan-cooled (cooling) tunnel (Dornier Manufacturing, Inc, Hartland, WI) was used to cool the co-extrudate material. The cooled co-extrudate was pelletized as described above using a Conair pelletizer (Conair Inc, Pittsburgh, PA) to produce pellets as shown in Figure 3.

The twin screws in the extruders of Figures 4 and 5 generally are operated at 314 and 264 rpm, respectively. These screw speeds may be varied, however, within a wide range, for example, from about 10 to about 1500 rpm, but preferably between 100 and 400 rpm.

In one exemplary composition, a solid dispersion composed of 42.5% troglitazone, 3.5% Tween 80, and 11.5% PEG 400 with an ethylcellulose outer coating layer was tested. The pelletized sample was 12 mm in length and 7 mm in diameter. Dissolution studies were carried out in 900 mL of dissolution medium (0.1 M phosphate buffer with 0.5% SLS, pH 8) maintained at 37°C using a USP dissolution apparatus II at 75 rpm. After introducing the sample into the dissolution medium, the solution was periodically sampled (10 mL aliquots) over 8 hours. The test samples were filtered through a Gelman Acrodisc® 0.45-µm filter pad (Pall Life Sciences, Ann Arbor, MI), and the filtrate was collected after discarding the first 2-mL portion. The samples were diluted by a factor of two (v/v) with methanol prior to testing. The extent of drug dissolved in the dissolution medium was determined using an HP 8453 diode array spectrophotometer at 284 nm.

The troglitazone core ethylcellulose coated samples were obtained by slicing portions from a long strand. The samples were coated, as described above, on all sides except on the cross-sectional faces. The integrity of the coating was maintained during the dissolution run. This sample was tested for percent dissolved and active ingredient (API) release rate. The core material appeared visually to erode and to dissolve from the cross-sectional exposed surface core. A linear dissolution profile (R = 0.99447) was obtained from this sample, indicating that an 8-hour, zero-order controlled-release profile was achieved with 100% release. This is graphically illustrated in Figure 6.

### EXAMPLE 2

By following the general procedure set forth in Example 1, a pellet comprising, an agent useful for treating Alzheimer's disease was prepared. The API was the compound 1-aza-bicyclo[2.2.1]heptan-3-one-[3-(3-methoxyphenyl)-prop-2-ynyl]-oxime, also referred to as CI-1017 (see United States Patent Number 5,346,911). Pellets having two different concentrations of components were prepared, as follows:

| API (% w/w) | PVP (% w/w) | PEG 400 (% w/w) |
|---|---|---|
| 17.6 | 70.4 | 12.0 |
| 72.8 | 18.2 | 9.0 |

In both cases, the twin-screw extruders for both the core and the coating were operated at 150 rpm. The rate of co-extrusion in both cases was 10 pounds per hour. The polyvinylpyrrolidone K-30 (PVP) and the API (CI-1017) were blended to uniformity for 20 minutes in a 16-quart V-blender. The coating rate was held constant at 1 pound per hour of Eudragit RSPO, containing 5% (w/w) triethylcitrate (TEC). The coating mixture was extruded using a melt temperature between about 157°C to about 161°C. The CI-1017 core was extruded at 100°C, and a melt pressure of 260 psi when the PEG level was 12% (w/w). A slightly lower pressure was used for the 9.0% level of PEG. The pellets of each concentration were uniform and dissolved uniformly over prolonged periods.

### EXAMPLE 3

By following the general procedures of Examples 1 and 2, the following co-extruded dosage forms of CI-1011 were prepared:

| Ingredients | | | Extrusion Rate |
|---|---|---|---|
| API (% w/w) | PVP (% w/w) | PEG 400 (%w/w) | (lbs/hr) |
| 17.0 | 68.1 | 14.9 | 5 |
| 17.3 | 69.1 | 13.6 | 7 |
| 17.3 | 69.1 | 13.6 | 10 |

CI-1011 is an agent useful for regulating lipids in mammals. It is now known generically as Avasimibe, and chemically is [(2,4,6-triisopropyl-phenyl)-acetyl]-sulfanic acid 2,6-diisopropyl phenyl ester (see United States Patent Number. 5,491,172). The pellets prepared with CI-1011 displayed uniform dissolution over prolonged periods.

### EXAMPLE 4

A formulation of CI-1027 was prepared according to the methods of Examples 1-3. CI-1027 is an agent useful for treating diabetes and for elevating HDL-cholesterol in mammals, and chemically is 6,6'-oxybis[2,2-dimethyl]-hexanoic acid (see U.S. Patent no. 5,783,600). The core for the CI-1027 blend was extruded at a melt temperature of 195°C, with a melt pressure of 1200 psi. A vacuum was applied to the vent (in barrel segment **310** in Figure 5) to reduce the number of bubbles that formed during the extrusion processing. The coating mixture was co-extruded, and the extrudate was sliced into pellets. The pellets had a composition of 17.6% (w/w) of CI-1027, 70.4% (w/w) PVP, and 12.0% (w/w) PEG 400. The co-extrusion was conducted so as to produce 10 pounds of extrudate per hour.

While this invention has been described with respect to specific embodiments thereof, it is not limited thereto. Nor is the present invention limited to the example shown.

## Claims

1. A pharmaceutical dosage form comprising a central core including a pharmaceutical agent in a controlled-release composition, said central core having two exposed opposite end surfaces and a peripheral surface at an outer edge of said core extending between said two opposed end surfaces, said peripheral edge surrounded by a diffusion-limiting sleeve, said sleeve being substantially impervious to water or bodily fluids, wherein said sleeve limits the diffusion of fluids into said central core, wherein said pharmaceutical dosage form is formed by co-extrusion of said central core and said sleeve resulting in said central core composed of a glassy matrix.

2. The pharmaceutical dosage form of claim 1, wherein said central core and said sleeve are generally cylindrical.

3. The pharmaceutical dosage form of claim 2, wherein the length of said sleeve is less than the diameter of said sleeve.

4. The pharmaceutical dosage form of claim 1, wherein said sleeve comprises at least one of ethylcellulose and polymethacrylate.

5. The pharmaceutical dosage form of claim 4, wherein said sleeve further includes a plasticizer.

6. The pharmaceutical dosage form of claim 5, wherein said plasticizer comprises at least one of triethylcitrate, dibutylsebacate, glycerin, and tributylcitrate.

7. The pharmaceutical dosage form of claim 1, wherein said central core comprises matrix materials comprising at least one material selected from polyethylene glycol, polyvinylalcohol, polymethacrylate, cellulose acetate phthalate, polyvinylpyrrolidone, hydroxypropylcellulose phthalate, hydroxypropylmethylcellulose phthalate, hydroxypropylmethylcellulose, hydroxyproplymethylcellulose acetate succinate, hydroxypropylcellulose, hydroxypropyethylcellulose, and polysorbate 80.

8. The pharmaceutical dosage form of claim 1, wherein said central core comprises polyvinylpyrrolidone and polyethylene glycol.

9. The pharmaceutical dosage form of claim 8, wherein said polyethylene glycol has a molecular weight in a range of about 400 to about 8000.

10. The pharmaceutical dosage form of claim 1, wherein said two exposed opposite end surfaces are planar and are parallel to each other.

11. A method of making a pharmaceutical dosage form having an outer layer and a central core, the method comprising:
feeding components of said outer layer into first extruder and feeding components of said central core into second extruder;
coextruding an indefinite length central core and outer layer from the second and first extruders respectively wherein the materials are melted or softened, to form a co-extrudate according to claim 1;
slicing said co-extrudate across the longitudinal axis thereof; and
cooling said co-extrudate to solidify the melted or softened materials.

12. The method of claim 11, wherein said co-extrudate is permitted to harden before said co-extrudate is sliced (cl14 + 9/30-31).

13. The method of claim 11, wherein said co-extrudate is sliced with a laser (10/1).

14. The method of claim 11, wherein said co-extrudate is heated to a temperature in a range of about 40°C to about 200°C (cl 14).

15. The method of claim 11, wherein the first and second extruders are twin-screw extruders ((9/13-14).

## Patentansprüche

1. Pharmazeutische Dosierungsform, umfassend einen zentralen Kern mit einem pharmazeutischen Mittel in einer Zusammensetzung mit kontrollierter Freisetzung, wobei der zentrale Kern zwei freiliegende gegenüberliegende Endflächen und eine Umfangsfläche an einem äußeren Rand des Kerns, der sich zwischen den beiden gegenüberliegenden Endflächen erstreckt, aufweist, wobei der Umfangsrand von einer diffusionsbeschränkenden Hülse umgeben ist, wobei die Hülse im wesentlichen gegenüber Wasser oder Körperflüssigkeiten undurchlässig ist, wobei die Hülse die Diffusion von Flüssigkeiten in den zentralen Kern beschränkt, wobei die pharmazeutische Dosierungsform durch Coextrusion des zentralen Kerns und der Hülse gebildet wird, was zu einem zentralen Kern, der aus einer glasartigen Matrix zusammengesetzt ist, führt.

2. Pharmazeutische Dosierungsform nach Anspruch 1, wobei der zentrale Kern und die Hülse im allgemeinen zylindrisch sind.

3. Pharmazeutische Dosierungsform nach Anspruch 2, wobei die Länge der Hülse geringer als der Durchmesser der Hülse ist.

4. Pharmazeutische Dosierungsform nach Anspruch 1, wobei die Hülse mindestens einen der Bestandteile Ethylcellulose und Polymethacrylat umfasst.

5. Pharmazeutische Dosierungsform nach Anspruch 4, wobei die Hülse ferner einen Weichmacher umfasst.

6. Pharmazeutische Dosierungsform nach Anspruch 5, wobei der Weichmacher mindestens einen der Bestandteile Triethylcitrat, Dibutylsebacat, Glycerin und Tributylcitrat umfasst.

7. Pharmazeutische Dosierungsform nach Anspruch 1, wobei der zentrale Kern Matrixmaterialien umfasst, die mindestens ein Material aus folgender Gruppe umfassen: Polyethylenglykol, Polyvinylalkohol, Polymethacrylat, Celluloseacetatphthalat, Polyvinylpyrrolidon, Hydroxypropylcellulosephthalat, Hydroxypropylmethylcellulosephthalat, Hydroxyproplymethylcellulose, Hydroxypropylmethylcelluloseacetosuccinat, Hydroxypropylcellulose, Hydroxypropylethylcellulose und Polysorbat 80.

8. Pharmazeutische Dosierungsform nach Anspruch 1, wobei der zentrale Kern Polyvinylpyrrolidon und Polyethylenglykol umfasst.

9. Pharmazeutische Dosierungsform nach Anspruch 8, wobei das Polyethylenglykol ein Molekulargewicht im Bereich von etwa 400 bis etwa 8 000 aufweist.

10. Pharmazeutische Dosierungsform nach Anspruch 1, wobei die zwei freiliegenden gegenüberliegenden Endflächen planar sind und parallel zueinander sind.

11. Verfahren zur Herstellung einer pharmazeutischen Dosierungsform mit einer äußeren Schicht und einem zentralen Kern, wobei das Verfahren folgendes umfasst:
das Zuführen von Komponenten für die äußere Schicht in einen ersten Extruder und das Zuführen von Komponenten für den zentralen Kern in einen zweiten Extruder;
das Coextrudieren eines zentralen Kerns unbestimmter Länge und einer äußeren Schicht aus dem ersten bzw. dem zweiten Extruder, wobei die Materialien geschmolzen oder erweicht werden, unter Bildung eines Coextrudats nach Anspruch 1;
das Aufschneiden des Coextrudats quer zu dessen Längsachse; und
das Abkühlen des Coextrudats, um die geschmolzenen oder erweichten Materialien zu verfestigen.

12. Verfahren nach Anspruch 11, wobei man das Coextrudat härten lässt, bevor es aufgeschnitten wird (cl14 + 9/30-31).

13. Verfahren nach Anspruch 11, wobei das Coextrudat mit einem Laser aufgeschnitten wird (10/1).

14. Verfahren nach Anspruch 11, wobei das Coextrudat auf eine Temperatur in einem Bereich von etwa 40 bis etwa 200 °C erwärmt wird (cl 14).

15. Verfahren nach Anspruch 11, wobei es sich beim ersten und beim zweiten Extruder um Doppelschneckenextruder handelt (9/13-14).

## Revendications

1. Forme de dosage pharmaceutique comprenant un noyau central contenant un agent pharmaceutique dans une composition à libération contrôlée, ledit noyau central ayant deux surfaces d'extrémité opposées exposées et une surface périphérique en un bord externe dudit noyau s'étendant entre lesdites deux surfaces d'extrémité opposées, ledit bord périphérique enveloppé par une enveloppe limitant la diffusion, ladite enveloppe étant sensiblement imperméable à l'eau ou aux fluides corporels, dans laquelle ladite enveloppe limite la diffusion des fluides dans ledit noyau central, dans laquelle ladite forme de dosage pharmaceutique est formée par co-extrusion dudit noyau central et de ladite enveloppe donnant ledit noyau central composé d'une matrice vitreuse.

2. Forme de dosage pharmaceutique selon la revendication 1, dans laquelle ledit noyau central et ladite enveloppe sont généralement cylindriques.

3. Forme de dosage pharmaceutique selon la revendication 2, dans laquelle la longueur de ladite enveloppe est inférieure au diamètre de ladite enveloppe.

4. Forme de dosage pharmaceutique selon la revendication 1, dans laquelle ladite enveloppe comprend au moins un composé choisi parmi l'éthylcellulose et le polyméthacrylate.

5. Forme de dosage pharmaceutique selon la revendication 4, dans laquelle ladite enveloppe comprend en outre un plastifiant.

6. Forme de dosage pharmaceutique selon la revendication 5, dans laquelle ledit plastifiant comprend au moins un composé choisi parmi le citrate de triéthyle, le sébacate de dibutyle, la glycérine et le citrate de tributyle.

7. Forme de dosage pharmaceutique selon la revendication 1, dans laquelle ledit noyau central comprenant des matrices comprenant au moins un composé choisi parmi le polyéthylène glycol, un alcool polyvinylique, le polyméthacrylate, le phtalate d'acétate de cellulose, la polyvinylpyrrolidone, le phtalate d'hydroxypropylcellulose, le phtalate d'hydroxypropylméthylcellulose, l'hydroxypropylméthylcellulose, le succinate d'acétate d'hydroxypropylméthylcellulose, l'hydroxypropylcellulose, l'hydroxypropyléthylcellulose et le polysorbate 80.

8. Forme de dosage pharmaceutique selon la revendication 1, dans laquelle ledit noyau central comprend la polyvinylpyrrolidone et le polyéthylène glycol.

9. Forme de dosage pharmaceutique selon la revendication 8, dans laquelle ledit polyéthylène glycol a un poids moléculaire compris dans la plage allant d'environ 400 à environ 8000.

10. Forme de dosage pharmaceutique selon la revendication 1, dans laquelle lesdites deux surfaces d'extrémité opposées exposées sont planaires et sont parallèles l'une par rapport à l'autre.

11. Procédé de préparation d'une forme de dosage pharmaceutique ayant une couche externe et un noyau central, le procédé comprenant les étapes consistant à :
alimenter les composants de ladite couche externe dans une première extrudeuse et alimenter les composants dudit noyau central dans une seconde extrudeuse,
co-extruder une longueur indéfinie du noyau central et de la couche externe à partir des première et seconde extrudeuses respectivement, dans lesquelles les matériaux sont fondus ou ramollis, pour former un produit co-extrudé selon la revendication 1 ;
découper ledit produit co-extrudé le long de son axe longitudinal ; et
refroidir ledit produit co-extrudé pour solidifier les matériaux fondus ou ramollis.

12. Procédé selon la revendication 11, dans lequel ledit produit co-extrudé est laissé durcir avant de découper ledit produit co-extrudé (cl14 + 9/30-31).

13. Procédé selon la revendication 11, dans lequel le produit co-extrudé est découpé au laser (10/1).

14. Procédé selon la revendication 11, dans lequel le produit co-extrudé est chauffé à une température comprise dans la plage allant d'environ 40 °C à environ 200 °C (cl 14).

15. Procédé selon la revendication 11, dans lequel les première et seconde extrudeuses sont des extrudeuses à double vis (9/13-14).
